# EUROPEAN PATENT SPECIFICATION

(11) **EP 1 309 333 B1**
(45) Date of publication and mention of the grant of the patent: **02.09.2009**
(21) Application number: 01960802.5
(22) Date of filing: 16.08.2001
(51) Int. Cl.: A61K 36/53, A61K 36/534, A61K 36/537, A61K 36/31, A61K 36/45, A61K 36/752, A61K 36/73, A61K 36/896, A61K 36/23, A61K 36/738, A61K 36/70, A61K 36/80, A61K 36/30, A61P 31/04

(54) **PLANT-DERIVED AND SYNTHETIC PHENOLIC COMPOUNDS AND PLANT EXTRACTS, EFFECTIVE IN THE TREATMENT AND PREVENTION OF CHLAMYDIAL INFECTIONS**
PFLANZLICHE UND SYNTHETISCHE PHENOLISCHE VERBINDUNGEN UND PFLANZENEXTRAKTE, WIRKSAM ZUR BEHANDLUNG UND PRÄVENTION VON CHLAMYDIENINFEKTIONEN
COMPOSES PHENOLIQUES DE SYNTHESE ET DERIVES DE PLANTES, ET EXTRAITS DE PLANTES PERMETTANT DE TRAITER ET DE PREVENIR LES INFECTIONS A CHLAMYDIA

(30) Priority: 17.08.2000 US 225735 P; 18.08.2000 FI 20001832
(43) Date of publication of application: 14.05.2003
(73) Proprietor: Control-Ox OY, 01490 Vantaa (FI)
(72) Inventor: VUORELA, Heikki, FIN-06100 Porvoo (FI); VUORELA, Pia, FIN-06100 Porvoo (FI); HILTUNEN, Raimo, FIN-00180 Helsinki (FI); LEINONEN, Maija, FIN-90100 Oulu (FI); SAIKKU, Pekka, FIN-00320 Helsinki (FI)
(74) Representative: Hakkila, Maini Annika
(86) International application number: PCT/FI2001/000726
(87) International publication number: WO 2002/014464

(56) References cited:
- EP-A1- 1 005 862
- US-A- 4 617 293
- US-A- 4 933 169
- DATABASE WPI Week 199731, Derwent Publications Ltd., London, GB; AN 1997-333515, XP002908053 & CN 1 106 682 A (CAI H) 16 August 1995

## Description

The invention relates to effective plant-derived phenolic compounds and to the corresponding synthetic compounds and plant extracts as well as compositions containing them, useful in the treatment and prevention of chlamydial infections. The plant-derived phenolic compounds and the corresponding synthetic compounds and plant extracts can be used in the preparation of pharmaceutical preparations.

Chlamydiae are small Gram-negative bacteria. Due to their unique intracellular reproduction cycle they have been classified as a separate order *Chlamydiales,* including genus *Chlamydia*. The genus *Chlamydia* was already initially divided into two species, *C. trachomatis* and *C. psittaci.* The division was based on biochemical properties:

| | *C. trachomatis* | *C. psittaci* |
|---|---|---|
| Accumulation of glycogen in chlamydial inclusions (iodine staining +) | + | - |
| Sensitivity to sulpha drugs | + | - |

From the beginning, "*C. trachomatis"* was considered a homogeneous group and "*C. psittaci*" a very heterogeneous group. When the chlamydial strain (later *C. pneumoniae*) causing respiratory infections without bird contacts was discovered, it was indisputably considered to belong to the group of "*C*. *psittaci"* because it satisfied the above-mentioned conditions based on biochemistry. *C*. *trachomatis* and *C. pneumonia* are different in respect of their surface structure. The main component of the surface structure of *C*. *trachomatis* is the major outer membrane protein (MOMP) that varies at four different sites and gives the basis on the division of *C. trachomatis* to, at the present, almost 20 different immunotypes. In *C. pneumoniae,* MOMP is very conservative and only one immunotype is found. In addition, the target cells in tissue are different: the epithelium of genitals and conjunctiva in the case of *C*. *trachomatis* and the epithelium of respiratory tract in the case of *C. pneumoniae.* The former, with the exception of rare lymphogranuloma venereum strain (LGV), is not capable of multiplying in phagocytes and macrophages, which specifically constitute the target cells of the latter. When penetrating into cells, *C. pneumoniae* uses a heparin receptor which is not used by genital chlamydiae with the exception of LGV. Furthermore, transmission routes and resulting clinical pictures are different: *C*. *pneumoniae* is transmitted via respiratory tract and may spread inside monocytes into the circulatory system, whereas *C. trachomatis* is transmitted principally in sexual contacts. In addition, the treatment is different: *C. trachomatis* is usually treated with a single dose, whereas for *C*. *pneumoniae,* even three-week antibiotic courses are recommended.

A list of diseases caused by or associated with these chlamydial species is shown below:

### C. trachoniatis:

- conjunctivitis
- cervicitis
- urethritis
- pelvic inflammatory disease (PID)
- infections of newborns e.g. infant pneumonitis
- peritonitis
- perihepatitis
- reactive arthritis

### C. pneumoniae:

- upper respiratory tract infections
- bronchitis
- pneumonia
- chronic obstructive pulmonary disease (COPD)
- asthma
- vasculitis
- atherosclerosis with its complications
- encephalitis
- certain types of multiple sclerosis
- part of the late onset of Alzheimer's disease

It was shown as early as 1989 that the chlamydial strain causing respiratory infections without bird contacts differed genetically both from *C. trachomatis* and from the described *C. psittaci* species so clearly that it was separated into its own species, *C. pneumoniae.* Its nucleic acid homology with *C*. *trachomatis* is below 10 %. *C. trachomatis* has extragenomic plasmids not found in human *C. pneumoniae* strains. The genomes of both species have been sequenced and the number of genes is considerably higher (about 200) in *C*. *pneumoniae* than in *C. trachomatis.* In a recent reclassification of chlamydiae, it has already been transferred to a totally different genus, *Chlamydophila.* Thus, there is good reason to believe that all that is known from *C. trachomatis* cannot be applied to *C*. *pneumoniae.*

Most common chlamydial species in humans are *C*. *pneumoniae* and *C. trachomatis* which cause common important diseases. *C. psittaci* is very widespread in the animal kingdom but can only occasionally also cause infections in man. Additionally *C*. *pecorum* is known causing infections in ruminants. The classification of new genera and species in order *Chlamydiales* is in progress.

*C. pneumoniae* is the most common chlamydiae of the mankind and almost everybody gets infected with it 2 to 3 times during the life time. *C. pneunioniae* can easily invade lung tissue and multiply in macrophages and endothelium of blood vessels. The clinical picture of respiratory infections caused by *C. pneumoniae* varies largely from the usually mild upper respiratory tract infections in children to serious pneumonias of adults. 5-10% of all pneumonias are caused by *C. pneumoniae. C. pneumoniae* is spread as an airway infection from people to people. Obviously some individuals are effective transmitters, because the infections become more common only at school age. In Nordic countries infections caused by *C. pneumoniae* occur as two to three years long epidemics with about six years' intervals.

Chlamydial infections are of incidious and latent nature and their chronic late complications are obviously most significant of all. Epidemiological studies indicate an important association between chronic *C. pneumoniae* infections and atherosclerosis: many studies have also revealed a connection between chlamydial infections and the incidence of acute myocardial infarction (AMI). Further, the chronic *C. pneumoniae* infection apparently plays a role in the outbreak of asthma as well as of chronic obstructive pulmonary disease.

Arteriosclerosis is a chronic inflammation state and *C*. *pneumonia* particles can be demonstrated in foam cells and smooth muscle cells in over half of the atherosclerotic plaques. In the AMI patients there often occurs an immune response to the chlamydial lipopolysaccharide (LPS), indicating the exacerbation of an infection. It has also been possible to detect chlamydiae in damaged heart valves and they are especially abundant in abdominal-aortic aneurysms. Additionally, *C*. *pneumoniae* have been discovered to play a role in cerebral infarcts and transient cerebral ischemic attacks. As yet it is not finally clear what role the chlamydiae found in the damaged site play in the development of the damage itself, but one factor in the slow progress of these diseases seems anyhow to be the chronic chlamydial infection. In animal models, however, *C*. *pneumoniae* has been shown to initiate and accelerate the development of the atherosclerosis. Epidemiological and clinical studies have shown that there is a clear connection between a chronic *C. pneumoniae* infection and atherosclerosis and AMI. In the latest studies, it has also been concluded that *C*. *pneumoniae* infection is a risk factor of the cardiac events. *C. pneumoniae* infection is often connected to cigarette smoking which obviously predisposes to a chronic chlamydial infection.

Antibiotic treatment has been observed to reduce the risk of heart attacks and it has also been possible to influence the common inflammation marker CRP and serum fibrinogen levels with antibiotic treatment. In most of the industrialised countries, the morbidity of heart diseases began to sink when the use of antibiotics, very effective against chlamydiae, became common in the treatment of other infections.

*C. trachomatis* is the most important cause of the genital infections of women. Additionally a part of the bacterial culture negative urinary tract infections of women in fertile age are caused by *C. trachomatis. C. trachomatis* is a common cause of the chronic endometriosis, and PID is the most common complication of *C. trachomatis* infections in women. A *C. trachomatis* infection can be almost symptomless, and even extrauterine pregnancy as well as infertility are known as complications of the obstructive scar formation caused by the incidious silent infection. About a half of the children born to chlamydiae carriers will get infected by the birth and about half of infected new-born children will develop inclusion conjunctivitis with *C. trachomatis* pneumonia as a complication. *C*. *trachomatis* also causes genital infections in men.

Chlamydiae are sensitive to tetracyclines and erythromycin; rifampicin and some new fluorokinolones are effective too. *C. trachomatis* is also, in contrast to e.g. *C. pneumoniae,* sensitive to sulpha drugs. In spite of the response to treatment, chlamydial infections are often recurring and there is also a risk that they become chronic. Chlamydiae multiply only inside the cell, and hence the new macrolide antibiotics and azalides concentrating efficiently into the cells are nowadays alternatives to tetracycline and erythromycin as a primary drug. In a complicated chlamydial infection the treatment possibly has to be continued for a long time and for example in Reiter's disease caused by chlamydiae, a three month's treatment is recommended.

Until now there are no vaccines for the prevention of chlamydial infections. The nature of the immune response is insufficiently known and a tendency towards hypersensitivity is connected to it.

In patent EP O 377 722 a method for the evaluation of the risk of a cardiac infarct, a method of diagnosing a heart and blood vessel disease as well as the use of drugs effective against chlamydiae are described. In this publication tetracyclines, erythromycin, rifampicillin and fluorokinolones are described as suitable drugs for the treatment or prevention of a chronic heart disease caused by chlamydiae.

WO 98/50074 describes, especially for the treatment of an infection caused by *C. pneumoniae,* a combination of anti-chlamydiae agents wherein the active ingredients are each effective at a certain stage of the life cycle of chlamydia.

In patent US 5 830 874 a method for diagnosing of arterial chlamydial granuloma caused by *C. pneumoniae,* as well as therapeutical compositions for the treatment of arterial chlamydial granulomatosis are described. As suitable therapeutically acting compounds tetracyclines, erythromycins, clarithromycins, azitromycin and kinolones etc. effective against chlamydiae are mentioned.

Patent JP 10 139 686 describes for the treatment of atherosclerosis caused by *C. pneumoniae* the use of 2-(3,4-dimetoxycinnamoyl)-aminobenzoic acid as a therapeutically active compound at a daily dosage of 100-1000 mag.

According to the above information there is an obvious need for new compounds and compositions which can be used in the treatment and prevention of chlamydial infections.

Shikimates or compounds formed from shikimic acid via a biosynthetic pathway, compounds formed via the acetate-malonate biosynthetic pathway and compounds formed via combinations of both pathways belong to the group of plant-derived phenolic compounds. Simple aromates, phenols, coumarins, lignans, lignins as well as flavonoids and their derivatives belong to said compounds. Simple aromates mainly include phenylpropane derivatives and phenylmethane derivatives. In nature flavonoids, which are structurally phenolic compounds, form a widespread plant pigment group. Flavonoids occur everywhere in the plant kingdom, in bryophytes, in the stonecrop family and in other lower plants. Most of all they have been found from higher plants and vascular plants and they occur in all fruits, vegetables and among others in tea as well as wines, especially red wines. Flavonoids occur in nature mainly in the glycosidic form, but they can also be free phenols and sulphates in the so-called aglycon form or as bound to polysaccharides and proteins. In most cases the flavonoids are of their chemical structure polyphenolic compounds. Over 8000 flavonoids have been identified from plants and they have a myriad of functions. They, due to their bitter taste, protect plants against noxious insects and, due to their antibiotic properties, protect plants against viruses and bacteria. According to the current opinion flavonoids are not nutritionally important compounds, but they seem to have beneficial effects on the health. This effect is apparently independent from the vitamins and minerals contained in the plant. Hardly anyone can avoid ingesting flavonoids, but their possibilities to effect depend, anyhow, on the absorption properties and bioavailability and additionally on the interaction of the simultaneously obtained flavonoids.

The antioxidant effect of natural phenolic compounds has been already known for quite a long time and the antioxidant effect as well as the capture of free radicals have been dealt with in several studies. According to the present research information the ability of flavonoids to prevent the oxidation of LDL cholesterol is considered as one of their most important properties. The oxidation of LDL cholesterol in the subendothelium of a blood vessel is the initial factor in atherogenesis. Many studies suggest that insufficient intake of flavonoids from the nutrition would be an important factor in the morbidity caused by the heart and blood vessel diseases. In human studies concerning flavonoids only a few most important flavonoids have been observed, of which quercetin has been shown to prevent the oxidation of LDL cholesterol and thus to reduce the risk of coronary disease, because the oxidised LDL cholesterol has clearly been related to the atherosclerotic stages.

The daily intake of flavonoids from the nutrition varies, for example according to a Dutch research, between 0-30 mg. In studies conducted in Finns, flavonoids have been noticed to show a modest protecting effect against the morbidity of heart and blood vessel diseases, but the differences in the intake of flavonoids on the other hand were rather small, the total amounts being about 2-6 mg/day. In a study carried out in Holland an inverse relation between from nutrition acquirable flavonols and flavanols and death cases caused by the heart and blood vessel diseases has been noticed. An inverse relation between the intake of flavonols and flavones and the risk of cardiac infarct has also been noticed. The flavonoids are further known to have an effect on inflammation and immune responses as well as on many other functions of the cell. Some flavonoids and many other phenolic natural compounds can prevent or enhance the calcium intake to the cell which is also demonstrated in Table 1 presented later.

The calcium channel blocking drugs have an important role in the treatment of heart and blood vessel diseases, such as chest pain caused by cardiac anoxia, of myocardial infarct, atherosclerosis and hypertension. These drugs act on the calcium channels by preventing the influx of calcium to the cell and thus enlarge the coronary artery as well as lower the peripheral resistance of blood vessels wherein the cardiac load is diminished. Large scale use of calcium blockers has lead e.g. to the development of screening programs in order to find the calcium channel blocking effect of compounds isolated from nature. As the screening medium in the studies e.g. a continuous cell line originating from a tumour of the posterior lobe of the pituitary gland of rat (GH₄C₁) has been used, as well as patch clamp technique, in which the separate calcium channels of one cell can be examined at a time. As the result of the studies naturally occurring compounds and extracts have been found which have calcium channel blocking or activating effects. These compounds have been found among plant-derived simple phenols, coumarins, flavonoids and extracts rich in said compounds. Some of these are in their blocking effect comparable with verapamil and some of the compounds have a tendency to enhance the calcium influx to the cell.

The present invention relates to the use of plant-derived phenolic compounds and the corresponding synthetic compounds and plant extracts containing them, in the manufacture of a medicament for the treatment and prevention of chlamydial infections, caused by Chlamydia pneumoniae.

The characteristic features of the use of the plant-derived phenolic compounds and the corresponding synthetic compounds and plant extracts containing them in the manufacture of medicaments useful in the treatment and prevention of chlamydial infections, are presented in the patent claims.

Surprisingly it has been found that certain plant-derived phenolic compounds, the corresponding synthetic compounds and plant extracts containing said plant-derived compounds have an antibiotic-like, strong effect against chlamydiae. The plant-derived phenolic compounds are phenolic compounds formed from shikimic acid via a biosynthetic pathway, phenolic compounds formed via the acetate-malonate pathway and phenolic compounds formed as a result of combinations of both pathways. These compounds, such as simple aromates, phenols, coumarins, lignans, lignins and flavonoids are obtained from products of the vegetable kingdom such as fruits and vegetables, especially citrus fruits, vegetables, berries, onions, tea, red wines etc.

The plant-derived phenolic compounds, the corresponding synthetic compounds and extracts containing them may be used as such or as mixtures of them, optionally in combination with sulphur compounds contained in garlic, such as alliicine or derivatives of alliicine.

The plant-derived natural phenolic compounds are flavonoids as well as phenylmethane and phenylpropane derivatives, phenolic acids, triterpenes, coumarins and cathecins, and extracts containing them, and also the corresponding synthetic compounds and pharmaceutically acceptable salts, esters and derivatives of the above cited compounds.

The compounds and extracts thereof have anti-chlamydial effect (inhition of formation of inclusions) of equal or more than 30% and preferable are the ones with the anti-chlamydial effect of equal or more than 90%, as defined in the examples. In the following are presented groups of preferred compounds and extracts:
- Flavones, such as apigenin, luteolin, flavone
- Flavonols, such as quercetin, rhamnetin, morin
- Flavanones, such as naringin
- Isoflavones, such as genistein
- Phenylmethane-derived compounds, such as methyl gallate, propyl gallate, octyl gallate, dodecyl gallate, isopropyl gallate
- Phenylpropane-derived compounds, such as:
   - Coumarins like umbelliferone, scopoletin, methoxy psoralen, xanthotoxin and coumarin
   - Flavan-3-ols like (-)-epigallocatechin, (-)-epicatechin, (+)-catechin and (-)-epicatechin gallate
- Synthetic compounds, such as flavonoids and coumarins like coumarin 106, 2'-methoxy-α-naphto-flavone, 6,2'-dirnethoxyflavone, 6-methylcoumarin, alpha-naphtoflavone, rotanone, 7-diethyl-amino-3-thenoylcoumarin
- Natural plant extracts, such as extracts of *Mentha longifolia, Mentha arvensis, Craleopsis speciosa, Salvia officinalis, Thymus vulgaris, Rumex acetocella, Rose rugosa, Veronica longifolia, Symphytum asperum, Artemisia vulgaris. Convallaria majalis, Quercus robur, Daucus carota, Fragaria iinumae, Brassica oleracea, Brassica napus, Medicago sativa, Citrus sinensis,* Phloem flour, *Vaccinum myriillus*

The plant-derived phenolic compounds, extracts containing them can conveniently be obtained from natural plants or parts of them by using any conventional technique for extracting and isolating substances. Braces, roots or leaves are suitably hydrodistilled and macerated or only hydrodistilled in order to obtain the desired extract, which may further be purified using any conventional purification technique known to a man skilled in the art. The corresponding synthetic compounds or their derivatives are usually commercially available substances or they may be manufacured using any known synthetic methods.

In the following Table 1 some of the preferred plant-derived phenolic compounds , the effect of the compounds on the calcium influx to the cell, the antioxidant effect as well as the chemical structure of the compounds are presented.

The chemical structures of the compounds presented in Table 1 are given in the following Schemes A, B, C, D and E. In Scheme A flavones, flavonols, in Scheme B flavanones, in Scheme C isoflavones, in Scheme D phenylmethane derivatives and in Scheme E phenylpropane derivatives are given. The substituents R₁₋₇ refer to the respective functional groups given in Table 1.

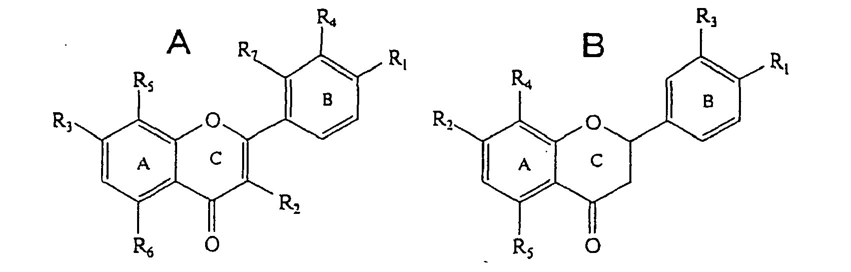

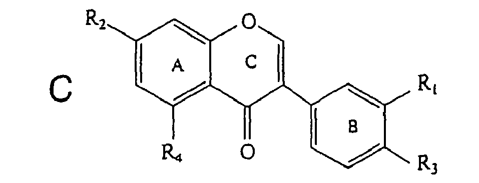

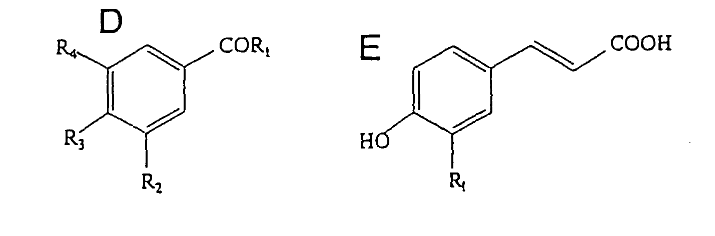

**Table 1**

| **Compound (20 µg/ml)** | **R₁** | **R₂** | **R₃** | **R₄** | **R₅** | **R₆** | **R₇** | **Effect on Ca²⁺ intake [%**] | **S.E.M** | **M_{w}** | **IC50 [moles L⁻¹] against DPPH** |
|---|---|---|---|---|---|---|---|---|---|---|---|
| **Flavones (Structure A)** | | | | | | | | | | | |
| Apigenin *^{a}* | OH | H | OH | H | H | OH | H | **- 29.3** | 4.0 | 270.2 | >3.70 x 10⁻³ |
| Luteolin *^{a}* | OH | H | OH | OH | H | OH | H | **- 51.4** | 7.7 | 286.2 | 1.20 x 10⁻⁵ |
| Acacetin *^{a}* | OCH₃ | H | OH | H | H | OH | H | **- 1.39** | 1.9 | 284.3 | >3.52 x 10⁻³ |
| Flavone *^{a}* | H | H | H | H | H | H | H | **-63.5** | 3.0 | 222.2 | >4.50 x 10⁻³ |
| Vitexin *^{a}* | OH | H | OH | H | Glu | OH | H | **- 2.12** | 5.1 | 432.4 | n.d. |
| Vitexin-2"-O-rhamnoside *^{a}* | OH | H | OH | H | GluRha | OH | H | **- 14.6** | 0.3 | 587.5 | n.d. |
| Luteolin-7-glucoside *^{a}* | OH | H | OGlu | OH | H | OH | H | **-16.3** | 1.3 | 448.4 | 1.07 x 10⁻⁵ |
| Luteolin-3',7-glucoside *^{a}* | OH | H | OGlu | Oglu | H | OH | H | **- 14.6** | 3.3 | 610.5 | n.d. |

| **Flavonol (Structure A)** | | | | | | | | | | | |
|---|---|---|---|---|---|---|---|---|---|---|---|
| Quercetin^{a} | OH | OH | OH | OH | H | OH | H | **+ 54.1** | 6.9 | 302.2 | 1.07 x 10⁻⁵ |
| Rhamnetin *^{a}* | OH | OH | OCH₃ | OH | H | OH | H | **+ 6.63** | 0.8 | 316.3 | 1.21 x 10⁻⁵ |
| Isorhamnetin *^{a}* | OH | OH | OH | OCH₃ | H | OH | H | **+52.4** | 2.3 | 316.3 | 1.81 x 10⁻⁵ |
| Morin *^{a}* | OH | OH | OH | H | H | OH | OH | **+ 48.0** | 5.9 | 302.2 | 2.39 x 10⁻⁵ |
| Quercitrin *^{a}* | OH | ORha | OH | OH | H | OH | H | **+ 20.1** | 0.6 | 448.4 | 1.29 x 10⁻⁵ |
| Rutin *^{a}* | OH | ORut | OH | OH | H | OH | H | **- 3.88** | 6.0 | 610.5 | 1.02 x 10⁻⁵ |

| **Flavanones (Structure B)** | | | | | | | | | | | |
|---|---|---|---|---|---|---|---|---|---|---|---|
| Naringenin *^{a}* | OH | OH | H | H | OH | - | - | **- 56.3** | 5.6 | 272.3 | >3.67 x 10⁻³) |
| Naringin *^{a}* | OH | ORhaGlu | H | H | OH | | - | **+ 6.5** | 7.5 | 580.5 | 7.30 x 10⁻³ |

| **Isoflavones (Structure C)** | | | | | | | | | | | |
|---|---|---|---|---|---|---|---|---|---|---|---|
| Daizein *^{a}* | H | OH | OH | H | - | - | - | **- 26.2** | 1.2 | 254.2 | >3.93 x 10⁻³ |
| Genistein *^{a}* | H | OH | OH | OH | - | - | - | **- 54.6** | 1.7 | 270.2 | >3.70 x 10⁻³ |
| Daizin *^{a}* H | OGlu | OH | H | - | - | - | - | **+ 7.6** | 5.9 | 416.4 | n.d. |
| Genistin *^{a}* | H | OGlu | OH | OH | - | - | - | **- 3.39** | 5.9 | 432.4 | n.d. |

| **Phenylmethanes (Structure D)** | | | | | | | | | | | |
|---|---|---|---|---|---|---|---|---|---|---|---|
| Benzoic acid *^{d}* | OH | H | H | H | - | - | - | **- 9.82** | 1.9 | 122.1 | >8.19 x 10⁻³ |
| Gallic acid *^{d}* | OH | OH | OH | OH | - | - | - | **- 5.33** | 1.3 | 170.1 | 2.15 x 10⁻⁵ |
| Syringic acid *^{a}* | OH | OCH₃ | OH | OCH₃ - | | - | - | **-10.9** | 4.0 | 198.2 | 3.26 x 10⁻⁵ |
| Methylgallate *^{b}* | OCH₃ | OH | OH | OH | - | - | - | **- 21.2** | 6.4 | 184.1 | 5.09 x 10⁻⁶ |
| Propylgallate *^{c}* | O(CH₂)₂CH₃ | OH | OH | OH | - | - | - | **- 37.9** | 4.1 | 212.2 | 1.33 x 10⁻⁵ |
| Octylgallate *^{c}* | O(CH₂)₇CH₃ | OH | OH | OH | - | - | - | **- 92.2** | 7.8 | 282.3 | 1.70 x 10⁻⁵ |
| Dodecylgallate *^{c}* | O(CH₂)₁₁CH₃ | OH | OH | OH | - | - | - | **- 40.4** | 6.8 | 338.4 | 1.54 x 10⁻⁵ |

| **Phenylpropanes (Structure E)** | | | | | | | | | | | |
|---|---|---|---|---|---|---|---|---|---|---|---|
| Caffeic acid *^{a}* | OH | - | - | - | - | - | - | **+ 9.71** | 2.4 | 180.2 | 2.14 x 10⁻⁵ |
| Ferulic acid *^{b}* | OCH₃ | - | - | - | - | - | - | **+ 9.23.** | 2.1 | 194.2 | 4.43 x 10⁻⁵ |

| | | | | | | | | | | | |
|---|---|---|---|---|---|---|---|---|---|---|---|
| Suppliers of the compounds: *^{a}* Roth, Germany; *^{b}* Sigma, MO, USA; *^{c}* Fluky, Switzerland; *^{d}* E. Merck, Germany M_{w}: The molecular weight of the compounds (g/moles) Abbreviations of the sugar moieties: Glu= Glucose, OGlu= Oglucose, ORha= Orhamnose , ORut= Orutinose, OGluRha= Oglucoserhamnose , ORhaGlu= Orhamnoseglucose n.d.: not determined | | | | | | | | | | | |

The anti-chlamydial effect of the plant-derived phenolic compounds quercetin, morin, rhamnetin and octyl gallate on *C. pneumoniae* and *C. trachomatis* was studied in examples 1-4. All of these compounds inhibited the growth of the *C. pneumoniae* in a concentration of 0.5-50 µg and quercetin, morin and rhamnetin were shown particularly effective on *C. trachomatis* when pretreated host cells were used. Preferable compounds for the treatment and prevention of a *C. pneumoniae* infection thus are for example phenolic compounds quercetin, morin, rhamnetin and octyl gallate isolated from natural materials, and for the treatment and prevention of a *C. trachomatis* infection in turn quercetin, morin and rhamnetin and extracts and partial fractions containing them.

The anti-chlamydial effect also of other plant-derived phenolic compounds, certain synthetic flavonoids and coumarins and mixtures of plant-derived phenolic compounds with garlic, using the concentration of 50 µm was studied in example 5 showing remarkable inhibiting effect. Correspondingly, the anti-chlamydial effect of alliicine, contained in garlic, on *C. pneumoniae* has been studied with similar results.

The anti-chlamydial effect of natural plant extracts and dietary plant extracts was studied in example 6 showing excellent inhibiting effect.

Preferred compounds and extracts are the ones with the anti-chlamydial effect (inhition of formation of inclusions) of equal or more than 30% and particularly preferable are the ones with the anti-chlamydial effect of equal or more than 90%, as defined in the examples.

A compound typically effective against chlamydia according to the present invention, is additionally an antioxidant and has an effect on the Ca²⁺ intake in the cell.

Octyl gallate is also a compound commonly used as a food additive.

As active ingredients the plant-derived phenolic compounds, the corresponding synthetic compounds and plant extracts, and mixtures of them may be dosed so that the daily supply counted as an aglycon is from 25 µg to 3000 mg. The plant-derived phenolic compounds and the corresponding synthetic compounds and plant extracts and mixtures of them may, according to the present invention, be prepared as pharmaceutical preparations in the form of capsules, tablets, ointments, liquid preparations or in other corresponding forms known to one skilled in the art. The preparations contain the active ingredient so that the daily supply is from 25 µg to 3000 mg counted as an aglycon, as unit doses preferably of from 25 µg to 500 mg.

The plant-derived phenolic compounds and the corresponding synthetic compounds and plant extracts and mixtures of them may also be added as such to food stuffs or they can be prepared as compositions suitable for food stuffs, such as herbal preparations, spices, granules or the like, which can be used as such, as added to the daily nourishment or functional food stuffs beneficial to health also called pro-health products, such as ready-prepared foods, porridges, salad dressings, drinks, milk-based products, edible fats, frozen products, freeze-dried food stuffs, speciality food stuffs, potato ships, dipping sauces etc. in connection with the production.

The plant-derived phenolic compounds and the corresponding synthetic compounds can exist in the compositions either in an aglycon form or in a glycosidic form.

The plant-derived phenolic compounds and the corresponding synthetic compounds and plant extracts and mixtures of them are safe as compounds. The compositions and preparations can be used both as a course of treatment of an acute chlamydial infection or by dosing the composition or preparation continuously and regularly with the daily nourishment in order to prevent a chlamydial infection. Because the chronic coronary heart disease causes considerably high costs for national economy, it is possible with the plant-derived phenolic compounds and the corresponding synthetic compounds and plant extracts and mixtures thereof and with compositions containing them to considerably prevent and slow down the upraise and outbreak of the heart and blood vessel diseases especially within the risk groups. The compositions and preparations can also be used for the treatment and prevention of an acute *C. trachomatis* infection as well as for the prevention of the late complications, such as infertility, extrauterine pregnancy and cervical cancer, and also for the treatment and prevention of other chlamydiae related infections and complications.

The invention is demonstrated in the following examples in more detail, but the invention is anyhow not restricted to the examples. In the examples, the direct anti-chlamydial effect of the compounds and plant extracts according to the invention on *C. pneumoniae* and *C. trachomatis* as well as the toxicity of the compounds and extracts towards the used host cells (HL cells, human lung tissue, standard diploid cell line) are described.

### Example 1

### The direct anti-chlamydial effect of plant-derived phenolic compounds (as inhibition of formation of inclusions) / C. pneumoniae K7 strain (clinical isolate)

**Results/ Concentration 50 µM :**

| Compounds Concentration 50 µM | Inhibition % of the DMSO control | Inhibition % of the DMSO control (pre-treated)^{*} |
|---|---|---|
| Quercetin | 90 | 90 |
| Morin | 99 | 96 |
| Rhamnetin | 99 | 59 |
| Octyl gallate | 100 | 100 |
| DMSO | 0 | 0 |

| | | |
|---|---|---|
| ^{*} The host cells were incubated for 1 day with the compound to be studied before infection. To 24-well plates containing host cells the compound to be studied in 1ml of maintenance medium was added in the same concentration as in the test procedure itself. The intention of this was to study the possible effect of the compound on the host cells themselves, which effect could be a factor inhibiting the infection. The test procedure was continued by the way described in the determination method. | | |

**Results/ Concentration 0.5 µM:**

| Compounds Concentration 0.5 µM | Inhibition % of the DMSO control | Inhibition % of the DMSO control (pre-treated)^{*} |
|---|---|---|
| Quercetin | 68 | 77 |
| Morin | 80 | 62 |
| Rhamnetin | 73 | 50 |
| Octyl gallate | 82 | 62 |
| DMSO | 0 | 0 |

| | | |
|---|---|---|
| * The host cells were incubated for 1 day with the compound to be studied before infection. To 24-well plates containing host cells the compound to be studied in 1ml of maintenance medium was added in the same concentration as in the test procedure itself. The intention of this was to study the possible effect of the compound on the host cells themselves, which effect could be a factor inhibiting the infection. The test procedure was continued by the way described in the determination method. | | |

### Example 2

### Inhibition of the infectivity / C. pneumonia

**Results/Concentration 50µM:**

| Compounds Concentration 50µM | Inhibition % of the DMSO control | Inhibition % of the DMSO control (pre-treated)^{*} |
|---|---|---|
| Quercetin | 76 | 0 |
| Morin | 94 | 76 |
| Rhamnetin | 100 | 67 |
| Octyl gallate | 100 | 100 |
| DMSO | 0 | 0 |

| | | |
|---|---|---|
| ^{*} The host cells were incubated for 1 day with the compound to be studied before infection. To 24-well plates containing host cells the compound to be studied in 1 ml of maintenance medium was added in the same concentration as in the test procedure itself. The intention of this was to study the possible effect of the compound on the host cells themselves, which effect could be a factor inhibiting the infection. The test procedure was continued by the way described in the determination method. | | |

**Results/Concentration 0.5µM:**

| Compounds 0.5 µM | Inhibition % of the DMSO control | Inhibition % of the DMSO control (pre-treated)^{*} |
|---|---|---|
| Quercetin | 58 | 0 |
| Morin | 81 | 53 |
| Rhamnetin | 100 | 75 |
| Octyl gallate | 59 | 42 |
| DMSO | 0 | 0 |

| | | |
|---|---|---|
| ^{*} The host cells were incubated for 1 day with the compound to be studied before infection. To 24-well plates containing host cells the compound to be studied in 1 ml of maintenance medium was added in the same concentration as in the test procedure itself. The intention of this was to study the possible effect of the compound on the host cells themselves, which effect could be a factor inhibiting the infection. The test procedure was continued by the way described in the determination method. | | |

### Example 3.

### Direct anti-chlamycial effect on C. trachomatis

*C. trachomatis,* cultivation in McCoy cells. The test procedure otherwise the same as with *C. pneumoniae.*

**Results/ Concentration 50µM:**

| Compounds Concentration 50µM | Inhibition % of the DMSO control | Inhibition % of the DMSO control (pre-treated)^{*} |
|---|---|---|
| Quercetin | 0 | 100 |
| Morin | 0 | 100 |
| Rhamnetin | 0 | 100 |
| Octyl gallate | 14 | 88 |

| | | |
|---|---|---|
| ^{*} The host cells were incubated for 1 day with the compound to be studied before infection. To 24-well plates containing host cells the compound to be studied in 1 ml of maintenance medium was added in the same concentration as in the test procedure itself. The intention of this was to study the possible effect of the compound on the host cells themselves, which effect could be a factor inhibiting the infection. The test procedure was continued by the way described in the determination method. | | |

**Results/ Concentration 0.5µM:**

| Compounds Concentration 0.5µM | Inhibition % of the DMSO control | Inhibition % of the DMSO control (pre-treated)^{*} |
|---|---|---|
| Quercetin | 0 | 20 |
| Morin | 0 | 100 |
| Rhamnetin | 0 | 17 |
| OG | 0 | 0 |

| | | |
|---|---|---|
| ^{*} The host cells were incubated for 1 day with the compound to be studied before infection. To 24-well plates containing host cells the compound to be studied in 1 ml of maintenance medium was added in the same concentration as in the test procedure itself. The intention of this was to study the possible effect of the compound on the host cells themselves, which effect could be a factor inhibiting the infection. The test procedure was continued by the way described in the determination method. | | |

### Example 4.

### Determination of the toxicity of some of the studied samples towards the host cells.

The determination was performed as in the previous tests but without any infection. The viability was determined with Trypan blue staining.

### Samples:

Q = quercetin
M = morin
R = rhamnetin
OG = octyl gallate
HL-C = HL cells in the sole nutrition medium
HL-CD = HL cells with a DMSO addition

| **TOXICITY TEST HL-C** | | | | | | | |
|---|---|---|---|---|---|---|---|
| Q50 | Q5 | Q0,5 | Q0,05 | Q0,005 | HL-C | HL-CD | µM |
| 10.5 | 9.2 | 7.0 | 11.7 | 7.4 | 19.7 | 9.6 | % |

| M50 | M5 | M0,5 | M0,05 | M0,005 | HL-C | HL-CD | µM |
|---|---|---|---|---|---|---|---|
| 11.2 | 8.3 | 8.4 | 9.0 | 8.3 | 19.0 | 9.0 | % |

| R50 | R5 | R0,5 | R0,05 | R0,005 | " | " | µM |
|---|---|---|---|---|---|---|---|
| 15.7 | 9.6 | 10.0 | 9.5 | 10.9 | | | % |

| OG50 | OG5 | OG0,5 | OG0,05 | OG0,005 | HL-C | HL-CD | µM |
|---|---|---|---|---|---|---|---|
| 16.4 | 8.8 | 7.9 | 7.4 | 8.0 | 5.7 | 7.8 | % |

| **TOXICITY TEST HL-C (pre-treated)** | | | | | | | |
|---|---|---|---|---|---|---|---|
| Q50 | Q5 | Q0,5 | Q0,05 | Q0,005 | | HL-CD | µM |
| 10.5 | 8.1 | 3.3 | 7.2 | 7.9 | | 30.1 | % |

| M50 | M5 | M0,5 | M0,05 | M0,005 | | " | µM |
|---|---|---|---|---|---|---|---|
| 9.7 | 12.7 | 6.4 | 8.1 | 14.4 | | | % |

| R50 | R5 | R0,5 | R0,05 | R0,005 | HL-C | HL-CD | µM |
|---|---|---|---|---|---|---|---|
| 16.9 | 8.0 | 10.6 | 7.6 | 6.6 | 10.8 | 10.7 | % |

| OG50 | OG5 | OG0,5 | OG0,05 | OG0,005 | HL-C | | µM |
|---|---|---|---|---|---|---|---|
| 14.8 | 8.5 | 5.0 | 7.1 | 7.0 | 7.9 | | % |

### Example 5.

### Direct anti-chlamydial effect of plant-derived phenolic compounds, certain synthetic compounds and mixtures on C. pneumoniae.

The concentration used was 50 µM.

| **Compound** | **Inhibition %** |
|---|---|
| 1. NATURAL FLAVONOIDS | |
| Apigenin | 100 |
| Luteolin | 100 |
| Flavone | 90 |
| Vitexin | 3 |
| Vitexin-2"-O-rhamnoside | 11 |
| Luteolin-7-glucoside | 23 |
| Luteolin-3',7-glucoside | 45 |
| Quercetin | 90 |
| Rhamnetin | 100 |
| Isorhamnetin | 70 |
| Morin | 100 |
| Quercitrin | 50 |
| Rutin | 46 |
| Naringenin | 16 |
| Naringin | 66 |
| Daidzein | 51 |
| Genistein | 60 |
| Daidzin | 0 |
| Genistin | 37 |
| Procyanidin B1 | 30 |
| Procyanidin B2 | 0 |
| | |

| 2. NATURAL PHENOLIC ACIDS | |
|---|---|
| Benzoic acid | 44 |
| Gallic acid | 27 |
| Syringic acid | 32 |
| Caffeic acid | 78 |
| Ferulic acid | 14 |
| Methyl gallate | 100 |
| Propyl Gallate | 100 |
| Octyl Gallate | 100 |
| Dodecyl gallate | 100 |
| | |

| 3. NATURAL TRITERPENE | |
|---|---|
| Resveratrole | 54 |
| | |

| 4. NATURAL COUMARINS AND CATHECINS | |
|---|---|
| Scopoletin | 96 |
| Methoxy psoralen | 100 |
| Umbelliferone | 75 |
| Xanthotoxin | 94 |
| Coumarin | 28 |
| (-)-Epicatechin gallate | 85 |
| (-)-Epigallocatechin | 58 |
| (-)-Epicatechin | 75 |
| (+)-Catechin | 76 |
| | |

| 5. SYNTHETIC FLAVONOIDS AND COUMARINS | |
|---|---|
| 3-(α-acetonylbenzyl)-4-hydroxycoumarin | 0 |
| Coumarin 102 | 63 |
| Coumarin 106 | 100 |
| 2'-methoxy-α-naphtoflavone | 100 |
| 6,2'-dimethoxyflavone | 73 |
| 6-methylcoumarin | 71 |
| Alpha-naphtoflavone | 92 |
| Rotenone | 100 |
| 7-diethylamino-3- thenoylcoumarin | 100 |
| 3-(2-benzoxazoyl)umbelliferone | 28 |
| Coumarin 30 | 50 |
| 3-benzoylbenzo(F)coumarin | 62 |
| 6,8-dibromocoumarin-3-carboxylic acid | 0 |
| 4-methyl-3-phenylcoumarin | 0 |

| 6. MIXTURES | |
|---|---|
| Octylgallat | 100 |
| Octylgallate 1/10 | 53 |
| Octylgallate 1/100 | 17 |
| Garlic | 26 |
| Garlic 1/10 | 25 |
| Garlic 1/100 | 19 |
| Octylgallate 50% + Garlic 50% | 100 |
| Octylgallate 50% + Garlic 50% 1/10 | 47 |
| Octylgallate 50% + Garlic 50% 1/100 | 34 |
| Octylgallate 50% + Garlic 50% 1/1000 | 6 |

### Example 6.

### Effect of plant extracts against C. pneumoniae

Initial screening of 101 extracts prepared from 61 natural and dietary plant materials against *C. pneumoniae* was conducted. The concentration used was 40 µg/well.

In the following are presented results of the selected most active natural plant extracts against *C. pneumoniae*, calculated from 4 different evaluations.

| **Plant** | **Family** | **Inhibition %** | **Viability** |
|---|---|---|---|
| N48. *Mentha longifolia* | Labiateae | 100 | OK |
| N53. *Mentha arvensis* | Labiateae | 100 | OK |
| *N44. Galeopsis speciosa* | Labiateae | 100 | OK |
| N57. *Salvia officinalis* | Labiateae | 100 | OK |
| N57. *Salvia officinalis* | Labiateae | 100 | OK |
| N58. *Thymus vulgaris* | Labiateae | 100 | OK |
| N34. *Rumex acetocella* | Polygonaceae | 100 | less |
| N30. *Rosa rugosa* | Rosaceae | 100 | OK |
| N28. *Veronica longifolia* | Scrophulariaceae | 100 | less |
| N8. *Symphytum asperum* | Boraginaceae | 100 | less |
| N22. *Artemisia vulgaris* | Asteraceae | 100 | OK |
| N37. *Convallaria majalis* | Convallariaceae | 100 | - |
| *Quercus robur* | Fagaceae | 100 | - |

In the following are presented results of the selected most active dietary plant extracts that showed 100% inhibition of *C*. *pneumoniae,* calculated from 4 different evaluations.

| **Plant** | **Family** | **Inhibition %** | **Viability** |
|---|---|---|---|
| D8. *Daucus carota* | Umbelliferae | 100 | - |
| D14. *Fragaria iinumae* | Rosaceae | 100 | less |
| D16. *Brassica oleracea* | Cruciferae | 100 | OK |
| D17. *Brassica napus* | Cruciferae | 100 | OK |
| D21. *Medicago sativa* | Leguminosae | 100 | OK |
| D23. *Citrus sinensis* | Rutaceae | 100 | Less |
| D25. Phloem flour | Polygonaceae | 100 | OK |
| D30. *Vaccinum myrtillus* | Ericaceae | 100 | OK |
| D31. *Vaccinum myrtillus* | Ericaceae | 100 | OK |

No significant activity differences were noticed between organically and normally grown plants.

In this study the extracts of plants that showed 100% inhibition of *C. pneumoniae* inclusions (n=4) were considered active. Five plants that belong to the family Labiateae were found paricularly active. Despite of the method of extraction, both hydrodistilled and macerated or only hydrodistilled extracts of *Salvia officinalis* were active against *C. pneumoniae.*

In the following is provided a description of the microbiological methods used.

### 1. CULTURE AND PASSAGE OF HL CELLS

Passage cultures of HL cells are made with intervals of 3 days. The host cells are inoculated on the day preceding the infection. The cells are rinsed with PBS 1 x 10 mls and harvested by trypsinisation (1:10, 1.5-2.0 ml/bottle; ca. 5 min in a laminar flow cabinet or 2 min in CO₂ at +37°C). The cell suspension is diluted to a level of ca. 350000 cells/ml of nutrition medium (RN). Cultivation at +37°C, CO₂ (5.0 %) and the RN medium changed 1-2 times a week. The HL cells can be freezed in liquid nitrogen [1 ml 7.5% FCS (RN) + 1 ml DMSO].

### 2. PURIFICATION OF THE CHLAMYDIAE

EB (Elementary Body) = the infective extracellular form of the chlamydiae. On the preceding day the necessary amount of the HL cell suspension infected with the chlamydia is taken to thawing. The cells are suspended well and kept in an ultrasonic bed for 2 minutes altogether [20 seconds of sonication (Amplitude is 24-25) and 10 seconds of cooling x 6]. The cells are broken and the chlamydiae remain undamaged.

The suspension is centrifuged for 10 minutes at 1600 rpm (550 x g), wherein the chlamydiaes are in the supernatant (the HL cells remnants are discarded). The supernatant is aspirated away and 5 ml of PBS is added, suspended and sonicated for 1 minute (10 seconds of sonication - 5 seconds of cooling x 6).

The chlamydiae can be stored frozen at -70°C.

### 3. CHLAMYDIAE TEST PROCEDURE

The cultivated HL cells are infected with *C. pneumoniae* EB's. The EB's which have invaded the cell are changed into the metabolically active reticulate body (RB) which are dividing by binary fission in the endosome vacuole or inclusion. After a certain time (about 72 hr) the *C. pneumoniae* RB's are condensing back into EB's, after which the inclusions are broken, the host cells are broken and the EB's are liberated. In this method the intention is to verify the inclusions before the breaking up of the host cells.

As the host cell of *C. pneumoniae* in *in vitro* conditions HL cells are used. As the host cells of *C*. *trachomatis* in *in vitro* conditions McCoy cells often are used.

### Infection of HL cells and McCoy cells

On the day preceding the infection the host cells are inoculated on a 24-well plate: the cells are harvested by trypsinisation, they are suspended in ca. 5 ml of the nutrition liquid and counted in a Burker's chamber. Each well are seeded using a concentration of 250000 - 400000 cells/well for cultivation. Before the addition of the cells to the well is if necessary round cover glass (diameter 13 mm) put for staining. Nutrition liquid is added so that the volume is 1 ml/well. On the following day the cells are infected with the desired bacterium.

Solution to be used in the infection and containing chlamydiae particles is mixed throughout. The old nutrition liquid is aspirated away. By the infection the inoculum which has been stored at -70°C is diluted to an IFU concentration of ca. 10³ so that the volume of the solution is at least 200 µl/well on a 24-well plate. The cells are infected by centrifuging at 1600 rpm (550 x g)/l hr. The nutrition medium is aspirated away and changed to a maintenance medium containing cyclohexamide and also containing the studied compound (DMSO concentration 0.2%), 1 ml/well. The cells are incubated in a 5% CO₂ atmosphere at +35°C. The cells infected with *C*. *pneumoniae* are incubated for 3 days. After 2-3 days the maintenance medium is removed and the cells washed with 1 x 1ml of PBS. To the wells 200 µl of SPG are added for further infection, harvested with a pipette tip and transferred into tubes. This is used for infecting new cells in the same way as above to test the inhibition of infectivity (chlamydiasidic effect).

### Chlamydial staining

The nutrition medium, which is above the cover glass left for staining, is aspirated away. The infected cells are fixed on the cover glass with methanol for 10 min. The cover glass is removed from the well and transferred onto a suitable fluorescein-conjugated monoclonal antibody on Parafilm in a moist chamber with the cell-containing side down. The cover glass is incubated for 30 min at +37°C and washed twice with PBS and once with water. Finally it is dried. The cover glass is put with the cell-containing side down on an object glass containing a fixative (e.g. Mounting medium). When viewed under the fluorescent microscope, cell culture specimens infacted with chlamydiae show a characteristic apple-green fluorescence of inclusions against a red counterstained background.

### Chemicals and reagents

RN = FCS nutrition medium: 100 ml of RPMI 1640 (Sigma), to which 3.5% L-glutamine and 10 mg streptomycin have been added (final concentration 20 µg/ml), 7.5 ml FCS. The ready solutions are stored at +8°C. Maintenance medium: 100 ml of FCS nutrition medium with addition of 50 µg of cyclohexamide final concentration 0.5 µg/ml. The ready solutions are stored at +8°C.

PBS (Dulbecco's Phosphate buffered saline, Gibco), pH 7.4

SPG = Saccharose 0.2M (37.5 g), KH₂PO₄ 3.8 mM (0.26 g), Na₂HPO₄ x 2H₂O 6.7 mM (0.61 g), glutamic acid (C₅H₉NO₄) 5 mM (0.36 g) as mixed to 500 ml of milli-Q water. Is after sterilisation stored at -20°C.

FCS, Foetal Calf Serum (Gibco, Scotland), is inactivated at 56°C, 30 min, filtered and stored at -70°C.

## Claims

1. Use of a plant-derived phenolic compound selected from apigenin, luteolin, quercetin, quercitrin, rhamnetin, isorhamnetin, morin, daidzein, genistein, caffeic acid, naringin, methyl gallate, octyl gallate, dodecyl gallate, isopropyl gallate, reservatrole, umbelliferone, scopoletin, methoxy psoralen and coumarin, or the corresponding synthetic compound, or a synthetic compound selected from coumarin 106, coumarin 102, 2'-methoxy-α-naphtoflavone, 6,2'-dimethoxy-flavone, 6-methylcoumarin, α-naphtoflavone, rotenone, coumarin 30, 3-benzoylbenzo-(F)coumarin and 7-diethyl-amino-3-thenoylcoumarin, or a natural plant extract selected from extract of *Mentha longifolia, Mentha arvensis, Galeopsis speciosa, Salvia officinalis, Thymus vulgaris, Rumex acetocella, Rosa rugosa, Veronica longifolia, Symphytum asperum, Artemisia vulgaris, Convallaria majalis, Quercus robur, Daucus carota, Fragaria iinumae, Brassica oleracea, Brassica napus, Medicago sariva, Citrus sinensis, Vaccinum myrtillus* or Phloem flour of *Polygonaceae,* or a mixture of them, in the manufacture of a medicament for the treatment and/or prevention of chlamydial infections caused by *Chlamydia pneumoniae.*

2. Use according to claim 1, **characterised in that** the anti-chlamydial effect on *Chlamydia pneumoniae* of the plant-derived phenolic compound or the extract or the synthetic compound, is equal or more than 30 % as inhibition of formation of inclusions.

3. Use according to claim 1 or 2, **characterised in that** the anti-chlamydial effect on *Chlamydia pneumoniae* of the plant-derived phenolic compound or the extract or the synthetic compound, is equal or more than 90 % as inhibition of formation of inclusions.

4. Use according to any one of claims 1 - 3, **characterised in that** the composition comprises a compound selected from apigenin, luteolin, quercetin, morin, rhamnetin, methyl gallate, octyl gallate, dodecyl gallate, isopropyl gallate, scopoletin, methoxy psoralen, coumarin 106, α-naphtoflavone, rotenone and 7-diethyl-amino-3-thenoylcoumarin.

5. Use according to any one of claims 1 - 4, **characterised in that** the composition contains 25 µg to 3000 mg, calculated as an aglycon, of the plant-derived phenolic compound or the synthetic compound, in the aglycon or glycosidic form or the extract.

6. Use according to any one of claims 1 - 5, **characterised in that** the compound is used in combination with sulphur compounds contained in garlic.

## Patentansprüche

1. Verwendung einer von Pflanzen stammenden phenolischen Verbindung, die ausgewählt ist aus Apigenin, Luteolin, Quercetin, Quercitrin, Rhamnetin, Isorhamnetin, Morin, Daidzein, Genistein, Kaffeesäure, Naringin, Methylgallat, Octylgallat, Dodecylgallat, Isopropylgallat, Reservatrol, Umbelliferon, Scopoletin, Methoxypsoralen und Cumarin, oder der entsprechenden synthetischen Verbindung, oder einer synthetischen Verbindung, die ausgewählt ist aus Cumarin 106, Cumarin 102, 2'-Methoxy-α-naphtoflavon, 6,2'-Dimethoxyflavon, 6-Methylcumarin, α-Naphtoflavon, Rotenon, Cumarin 30, 3-Benzoylbenzo[f]-cumarin und 7-Diethyl-amino-3-thenoylcumarin, oder eines natürlichen Pflanzenextrakts, der ausgewählt ist aus Extrakt von *Mentha longifolia, Mentha arvensis, Galeopsis speciosa, Salvia officinalis, Thymus vulgaris, Rumex acetocella, Rosa rugosa, Veronica longifolia, Symphytum asperum, Artemisia vulgaris, Convallaria majalis, Quercus robur, Daucus carota, Fragaria iinumae, Brassica oleracea, Brassica napus, Medicago sativa, Citrus sinensis, Vaccinum myrtillus* oder Phloemmehl von *Polygonaceae*, oder einer Mischung davon zur Herstellung eines Medikaments zur Behandlung und/oder Prävention von Chlamydieninfektionen, die durch *Chlamydia pneumoniae* verursacht sind.

2. Verwendung nach Anspruch 1, **dadurch gekennzeichnet, dass** die gegen Chlamydien gerichtete Wirkung der von Pflanzen stammenden phenolischen Verbindung oder des Extrakts oder der synthetischen Verbindung auf *Chlamydia pneumoniae* gleich oder mehr als 30 % als Hemmung der Bildung von Einschlüssen ist.

3. Verwendung nach Anspruch 1 oder 2, **dadurch gekennzeichnet, dass** die gegen Chlamydien gerichtete Wirkung der von Pflanzen stammenden phenolischen Verbindung oder des Extrakts oder der synthetischen Verbindung auf *Chlamydia pneumoniae* gleich oder mehr als 90 % als Hemmung der Bildung von Einschlüssen ist.

4. Verwendung nach einem der Ansprüche 1 bis 3, **dadurch gekennzeichnet, dass** die Zusammensetzung eine aus Apigenin, Luteolin, Quercetin, Morin, Rhamnetin, Methylgallat, Octylgallat, Dodecylgallat, Isopropylgallat, Scopoletin, Methoxypsoralen, Cumarin 106, α-Naphtoflavon, Rotenon und 7-Diethyl-amino-3-thenoylcumarin ausgewählte Verbindung aufweist.

5. Verwendung nach einem der Ansprüche 1 bis 4, **dadurch gekennzeichnet, dass** die Zusammensetzung 25 µg bis 3000 mg, berechnet als Aglykon, der von Pflanzen stammenden phenolischen Verbindung oder der synthetischen Verbindung in Form des Aglykons oder in glykosidischer Form oder Extrakt aufweist.

6. Verwendung nach einem der Ansprüche 1 bis 5, **dadurch gekennzeichnet, dass** die Verbindung in Kombination mit in Knoblauch enthaltenen Schwefelverbindungen verwendet wird.

## Revendications

1. Utilisation d'un composé phénolique tiré de plantes, choisi parmi apigénine, lutéoline, quercétine, quercitrine, rhamnétine, isorhamnétine, morine, daidzéine, genistéine, acide caféique, maringine, gallate de méthyle, gallate d'octyle, gallate de dodécyle, gallate d'isopropyle, réservatrole, umbelliférone, scopolétine, méthoxy psoralène et coumarine, ou du composé de synthèse correspondant, ou d'un composé de synthèse choisi parmi coumarine 106, coumarine 102, 2'-methoxy-α-naphtoflavone, 6,2'-dimethoxyflavone, 6-méthylcoumarine, α-naphto-flavone, roténone, coumarine 30, 3-benzoylbenzo-(f)-coumarine et 7-diéthyl-amino-3-thénoylcoumarine, ou d'un extrait de plantes naturel choisi parmi un extrait de *Mentha longifolia, Mentha arvensis, Galeopsis speciosa, Salvia officinalis, Thymus vulgaris, Rumex acetocella, Rosa rugosa, Veronica longifolia, Symphytum asperum, Artemisia vulgaris, Convallaria majalis, Quercus robur, Daucus carota, Fragaria iinumae, Brassica oleracea, Brassica napus, Medicago sativa, Citrus sinensis, Vaccinum myrtillus* ou de farine de phloème de *Polygonaceae,* ou d'un mélange de ceux-ci, dans la fabrication d'un médicament pour le traitement et/ou la prévention d'infections à chlamydia causées par *Chlamydia pneumoniae.*

2. Utilisation selon la revendication 1,
**caractérisée en ce que** l'effet anti-chlamydia, sous la forme d'une inhibition de la formation d'inclusions, sur *Chlamydia pneumoniae* du composé phénolique tiré de plantes ou de l'extrait, ou du composé de synthèse, est égal ou supérieur à 30%.

3. Utilisation selon la revendication 1 ou 2,
**caractérisée en ce que** l'effet anti-chlamydia, sous la forme d'une inhibition de la formation d'inclusions, sur *Chlamydia pneumoniae* du composé phénolique tiré de plantes ou de l'extrait, ou du composé de synthèse, est égal ou supérieur à 90%.

4. Utilisation selon l'une quelconque des revendications 1-3, **caractérisée en ce que** la composition comprend un composé choisi parmi apigénine, lutéoline, quercétine, morine, rhamnétine, gallate de méthyle, gallate d'octyle, gallate de dodécyle, gallate d'isopropyle, scopolétine, méthoxy psoralène, coumarine 106, α-naphtoflavone, roténone et 7-diéthyl-amino-3-thénoylcoumarine.

5. Utilisation selon l'une quelconque des revendications 1-4, **caractérisée en ce que** la composition contient 25 µg à 3000 mg, calculé en tant qu'aglycone, du composé phénolique tiré de plantes ou du composé de synthèse, sous la forme aglycone ou glycoside, ou de l'extrait.

6. Utilisation selon l'une quelconque des revendications 1-5, **caractérisée en ce que** le composé est utilisé en combinaison avec des composés de soufre contenu dans l'ail.
